# EUROPÄISCHE PATENTANMELDUNG

(11) **EP 1 088 808 A1**
(43) Veröffentlichungstag der Anmeldung: **04.04.2001**
(21) Anmeldenummer: 00117402.8
(22) Anmeldetag: 11.08.2000
(51) Int. Cl.: C07C 37/08, C07C 45/53

(54) **Verfahren zur Herstellung von Phenol, Aceton und Cyclohexanon**

(30) Priorität: 30.09.1999 DE 19946886
(71) Anmelder: Phenolchemie GmbH & Co. KG, 45966 Gladbeck (DE)
(72) Erfinder: van Barnefeld, Heinrich, Dr., 46244 Bottrop (DE); Pompetzki, Werner, Dr., 46284 Dorsten (DE); Gerlich, Otto, Dr., 45966 Gladbeck (DE); Kleinloh, Werner, Dr., 45721 Haltern (DE)
(74) Vertreter: Olbricht, Gerhard, Dr.

(57) **Zusammenfassung**

Phenol wird überwiegend durch Hocksche Spaltung von Cumolhydroperoxid hergestellt, wobei als Koppelprodukt zwangsläufig zu gleichen molaren Teilen Aceton anfällt, für welches in Zukunft aber geringere Absatzmärkte als für Phenol prognostiziert werden.

Erfindungsgemäß wird Phenol durch Oxidation eines Cumol und Cyclohexylbenzol im Gewichtsverhältnis von mindestens 3 : 1 enthaltenden Gemischs und anschließende Hocksche Spaltung hergestellt, so daß über die Zusammensetzung des Eduktgemisches das Verhältnis der Wertprodukte Phenol : Aceton : Cyclohexanon gesteuert derart werden kann, daß Überkapazitäten einer der beiden Carbonylverbindungen vermieden werden.

Herstellung von Phenol, Aceton und Cyclohexanon.

## Beschreibung

Die Erfindung betrifft ein Verfahren zur Herstellung von Phenol durch Hocksche Spaltung von Arylhydroperoxiden. Unter Arylhydroperoxiden werden im Rahmen dieser Erfindung alle Hydroperoxide verstanden, bei deren Hockscher Spaltung Phenol gebildet wird.

Phenol ist ein wichtiger chemischer Grundstoff mit breiter Anwendungspalette. Neben seiner Verwendung als Lösemittel wird Phenol unter anderem zur Herstellung von Phenol-Harzen, Bisphenol-A, ε-Caprolactam, Adipinsäure, Alkylphenolen und Weichinachern eingesetzt.

Es ist bekannt, Phenol durch Hocksche Spaltung eines geeigneten Hydroperoxids herzustellen. Dabei entsteht neben dem Phenol als Hydroxy-Verbindung stets als Koppelprodukt eine Carbonyl-Verbindung, die aus wirtschaftlichen Gründen ebenfalls einer geeigneten Verwertung zugeführt werden können muß.

Die Patentschrift FR-1 030 020 lehrt die Oxidation von Cyclohexylbenzol mit Sauerstoff oder Luft zu Cyclohexylbenzolhydroperoxid und dessen anschließende Hocksche Spaltung zu Phenol und Cyclohexanon. Cyclohexanon ist ebenfalls ein interessantes Wertprodukt, das zum Beispiel als Lösemittel oder zur Herstellung von Adipinsäure und Cyclohexanonoxim, das für die Herstellung von ε-Caprolactam benötigt wird, verwendet werden kann.

In der weiteren Patentliteratur zu diesem Verfahren wird die Oxidation von Cyclohexylbenzol mit Luft oder Sauerstoff zum Cyclohexylbenzolhydroperoxid bei Temperaturen zwischen 60 °C und 200 °C unter Normal- oder Überdruck mit und ohne Katalysator beschrieben. Während in der Patentschrift EP-0 037 167 der Einsatz von gradkettigen C₁₄- bis C₁₆-Alkylaminen oder von Polyvinylpyrrolidon als Katalysator für die Cyclohexylbenzoloxidation beschrieben wird, wird in den Patentdokumenten FR-1 030 020 und BE-551 908 die Oxidation auch ohne Katalysator dargestellt. US-Patent 4 299 991 beschreibt die Oxidation von Cyclohexylbenzol in Gegenwart eines Polymaleinimid-Katalysators.

Die Spaltung des durch Oxidation von Cyclohexylbenzol gewonnenen Cyclohexylbenzolhydroperoxids zu Phenol und Cyclohexanon kann nach FR-1 030 020 mit Cyclohexylbenzol als Lösemittel säurekatalysiert mittels Benzolsulfonsäure oder Schwefelsäure erfolgen. Nach US-Patent 4 246 203 läßt sich die Spaltung von Cyclobexylbenzolhydroperoxid auch bei Temperaturen von 120 bis 200 °C in Gegenwart von 50 bis 2 000 ppm Schwefelsäure durchführen, wobei die freigesetzte Reaktionswärme direkt zur Verdampfung des Phenols und des Cyclohexanons genutzt wird. Eine Reihe neuerer Patentdokumente der Fa. Philips beschreibt im Vergleich zu wäßrigen Säurekatalysatoren verbesserte Spalt-Katalysatoren, die den Nachteil der mit den Säurekatalysatoren stets verbundenen anschließenden Neutralisation des Produktstroms, die zusätzlich dessen weitere Aufarbeitung beeinträchtigen kann, vermeiden. So lehrt US-Patent 4 487 970 den Einsatz von SbF₅ und Graphit als Katalysator, während US-Patent 4 480 141 stattdessen BF₃ x H₃PO₄ und US-Patent 4 482 757 ein organisches Phosphoniumhalogenid der Formel R₄Pₓ und eine Säure wie Salzsäure, Schwefelsäure oder Phosphorsäure verwenden. US-Patent 4 532 360 lehrt schließlich ein direktes einstufiges Verfahren zur Herstellung von z. B. Phenol und Cyclohexanon aus Cyclohexylbenzol in Gegenwart von Bromwasserstoff oder Chlorwasserstoff und mindestens einem Additiv aus der Gruppe Ceriumoxid, Triphenylborat, Bortriphosphat und Wasser.

Phenol wird heutzutage jedoch in der Hauptsache durch Hocksche Spaltung von Cumolhydroperoxid hergestellt. Bei diesem sogenannten Cumolverfahren wird zunächst Cumol mit vorzugsweise Luft oder Sauerstoff zum Cumolhydroperoxid oxidiert, welches - nach üblicherweise einer destillativen Aufkonzentrierung durch Abtrennung von nicht umgesetztem Cumol auf einen Cumolhydroperoxidgehalt von 60 bis 85 Gew.-% - anschließend unter Säurekatalyse mit vorzugsweise Schwefelsäure zu Phenol und Aceton gespalten wird. Einen guten Überblick über das Cumolverfahren geben z. B. Weissermel/Arpe, Industrielle Organische Chemie, 2. Auflage, Verlag Chemie, 1978, oder Ullmann's Encyclopedia of Industrial Chemistry, Vol. A 19, S. 302 ff., VCH Verlagsgesellschaft, 1991. Neuere Weiterentwicklungen des Cumolverfahrens betreffen vor allem den Bereich der Cumolhydroperoxid-Spaltung und der Aufarbeitung des Spaltproduktes zur Verringerung der Nebenproduktbildung und des Energieverbrauchs, vgl. z. B. EP-0 589 588 A1, EP-0 670 296 A1 oder WO 97/06905.

Das Cumolverfahren führt stets molar zu gleichen Teilen zur Gewinnung von Phenol und Aceton. Bezogen auf das Gewicht fallen pro Tonne Phenol 0,62 Tonnen Aceton an. Ein wesentlicher Verwendungszweck für beide Produkte ist die Bisphenol-A-Synthese, wobei entsprechend der Reaktionsgleichung ausgehend von 2 mol Phenol und 1 mol Aceton 1 mol Bisphenol-A erhalten wird. Bisphenol-A dient als Ausgangsstoff zur Herstellung von Polycarbonaten und Epoxidharzen und wird daher in großen Mengen produziert. Zur Synthese von Bisphenol-A wird aber - molar betrachtet - nur halb so viel Aceton wie Phenol benötigt. Zwar besitzt auch Aceton weitere Verwendungsmöglichkeiten, z. B. als Läsemittel oder zur Herstellung von z. B. Methylmethacrylat, doch wird insgesamt ein unterschiedliches Marktwachstum für Phenol und Aceton in der Weise prognostiziert, daß molar etwas mehr Phenol als Aceton benötigt werden wird. Diesem unterschiedlichen Marktbedürfnis vermag das Cumolverfahren aufgrund seines Reaktionsschemas jedoch nicht zu entsprechen.

Damit stellt sich die Aufgabe, ein Verfahren zur Herstellung von Phenol bereitzustellen, das zwar ebenfalls Aceton erzeugt, aber auf wirtschaftliche Weise eine flexible Anpassung der Produktion an eine molar betrachtet höhere Phenol- als Acetonnachfrage erlaubt.
Diese Aufgabe wird erfindungsgemäß gelöst gemaß Patentanspruch 1 durch ein Verfahren zur Herstellung von Phenol durch Hocksche Spaltung von Arylhydroperoxiden, das dadurch gekennzeichnet ist, daß ein Gemisch, das Cumol und Cyclohexylbenzol in einem Gewichtsverhältnis von Cumol zu Cyclohexylbenzol von mindestens 3 : 1 enthält, mit molekularen Sauerstoff enthaltendem Gas oxidiert und dabei entstandenes Cumolhydroperoxid und Cyclohexylbenzolhydroperoxid zu Phenol, Aceton und Cyclohexanon gespalten wird.

Überraschenderweise wurde gefunden, daß mit Hilfe der aus dem Cumolverfahren bekannten Verfahrensschritte nicht nur auch Cyclohexylbenzol behandelt und zu Phenol und Cyclohexanon umgesetzt werden kann, sondern daß auch bestimmte Gemische, die gleichzeitig Cumol und Cyclohexylbenzol enthalten, als Edukt verwendet und somit gemeinsam zu Phenol, Aceton und Cyclohexanon umgesetzt werden können. Damit kann durch Anpassung der Zusammensetzung des Eduktgemisches in bezug auf den Gehalt an Cumol und Cyclohexylbenzol das molare Verhältnis von Phenol :Aceton: Cyclohexanon im Produktgemisch gezielt gesteuert werden und in der Regel ein nicht zu vermarktender Überschuß an einer der beiden entstehenden Carbonylverbindungen vermieden werden. Das erfindungsgemäße Verfahren bietet damit den wesentlichen Vorteil, die Produktion an die Märkte für die Wertprodukte Phenol, Aceton und Cyclohexanon anpassen zu können, wobei insbesondere die prognostizierte Aceton-Überkapazität vermieden werden kann.

Das erfindungsgemäße Verfahren bietet darüber hinaus den Vorteil, daß die im Vergleich zur Oxidation von reinem Cumol langsamere Reaktionsgeschwindigkeit bei der Oxidation von reinem Cyclohexylbenzol durch die Verwendung von Cumol und Cyclohexylbenzol enthaltenden Eduktgemischen positiv beeinflußt wird.

Da die erfindungsgemäße Umsetzung des Cumol und Cyclohexylbenzol enthaltenden Gemischs unter den aus dem herkömmlichen Cumolverfahren bekannten Bedingungen erfolgen kann, ist das erfindungsgemäße Verfahren zudem schnell und einfach realisierbar. Vorzugsweise wird bei der Realisierung im Bereich der Oxidation und der Spaltung auf bestehende Anlagenteile zurückgegriffen; lediglich bei der Zuführung der Edukte und insbesondere bei der Aufarbeitung des Spaltproduktstroms zu den einzelnen Wertprodukten sind Anpassungen erforderlich. Eine bestehende, nach dem herkömmlichen Cumolverfahren arbeitende Produktionsanlage für Phenol und Aceton kann daher leicht so erweitert werden, daß sie zur Durchführung des erfindungsgemäßen Verfahrens geeignet ist, und je nach Marktsituation erfindungsgemäß oder nur zur Herstellung von Phenol und Aceton betrieben werden.

Als Edukt für das erfindungsgemäße Verfahren dient ein Gemisch, das ein Gewichtsverhältnis von Cumol zu Cyclohexylbenzol von mindestens 3 : 1, vorzugsweise von 5 : 1 bis 9 : 1, aufweist. Dieses Gemisch kann bereits in Lagertanks vorgehalten werden oder durch Vermischen aus getrennten Quellen erst vor dem Eintritt in die Oxidationsreaktoren gebildet werden. Es ist auch möglich, Cumol oder Cyclohexylbenzol enthaltende Stoffströme getrennt in die Oxidationsreaktoren einzudosieren und dort zu vermischen. Als Oxidationsreaktoren dienen vorzugsweise die vom Cumolverfahren bekannten Blasensäulenreaktoren. Die Oxidation erfolgt analog zum Cumolverfahren vorzugsweise ohne Katalysator bei Temperaturen von 100 °C bis 140 °C und Drücken von 1 bis 20 bar absolut in Gegenwart von bevorzugt Luft oder Sauerstoff als molekularen Sauerstoff enthaltendem Gas, bis in der Regel ein Gesamtgehait von maximal 35 Gew.-% an Peroxiden im Produktstrom aus der Oxidation erreicht ist. Der Rest umfaßt überwiegend nicht umgesetztes Cumol und Cyclohexylbenzol, da vorzugsweise ein weitestgehend reines Cumol/Cyclohexylbenzol-Gemisch eingesetzt wird. Ferner sind in geringen Mengen einige bei der Oxidation gebildete Nebenprodukte enthalten. Hierzu zählen insbesondere Dimethylphenylcarbinol, Phenylcyclohexanol-1, Acetophenon und Caprophenon.
Wie beim herkömmlichen Cumolverfahren kann der Produktstrom aus der Oxidalion direkt oder über eine Vorlage als Zwischenspeicher einer Konzentrierungseinheit zugefürt werden, in der vorzugsweise destillativ unter Vakuum durch Abtrennung von nicht umgesetztem Cumol und/oder Cyclohexylbenzol der Gehalt an Cumolhydroperoxid bevorzugt auf 30 bis 60 Gew.-% und/oder an Cyclohexylbenzolhydroperoxid auf bevorzugt 5 bis 20 Gew.-% im Stoffstrom erhöht wird. Das abgetrennte Cumol und/oder Cyclohexylberizol wird vorzugsweise ggf nach weiterer Aufbereitung zur Oxidation zurückgeführt.

Der Stoffstrom aus der Aufkonzentrierung wird nun der Spaltung zugeführt, die erfindungsgemäß bevorzugt säurekatalysiert, vorzugsweise mittels Schwefelsäure, in homogener Phase erfolgt. Cumolhydroperoxid und Cyclohexylbenzolhydroperoxid werden hier im wesentlichen zu Phenol, Aceton und Cyclohexanon umgesetzt. Geeignete Spaltreaktoren und Reaktionsbedingungen sind z. B. aus EP-0 589 588 A1 oder WO 97/06905 bekannt. Es kann vorteilhaft sein, das Spaltprodukt analog zum Verfahren in diesen Patentdokumenten einer Nachtemperung zur Verringerung des Gehalts an unerwünschten, die Ausbeute verringenden Nebenprodukten zu unterziehen. Anschließend erfolgt eine vorzugsweise destillative Aufarbeitung des Spaltproduktgemisches auf dem Fachmann vertraute, zum Cumolverfahren analoge Weise, um die Wertprodukte Phenol, Aceton und Cyclohexanon zu isolieren.

Das erfindungsgemäße Verfahren ist nicht auf die hier skizzierten Hauptverfahrensschritte beschränkt; vielmehr können alle aus dem Cumolverfahren bekannten Verfahrensvarianten, Ergänzungen oder Verschaltungen, insbesondere wenn sie darauf abzielen, die Nebenproduktbildung zu verringern und den Energieverbrauch zu optimieren, auch auf das erfindungsgemäße Verfahren übertragen werden. In Kenntnis des erfindungsgemäßen Verfahrens erschließen sich dem Fachmann vor dem Hintergrund des Cumolverfahrens vielfältige verfahrenstechnische und apparative Ausgestaltungen.

Mit Hilfe des erfindungsgemäßen Verfahrens lassen sich Spaltausbeuten an Phenol, Aceton und Cyclohexanon von über 90 % erreichen. Die Zusammensetzung des in der Oxidation eingesetzten Eduktgemisches wird hinsichtlich seines Gehalts an Cyclohexylbenzol bevorzugt derart angepaßt, daß für die Wertprodukte Aceton und Cyclohexanon, die als Koppelprodukte zum Phenol anfallen, jeweils ausreichende Absatzmöglichkeiten bestehen. Auf diese Weise werden Verluste durch Überkapazitäten vermieden und eine hohe Wirtschaftlichkeit erreicht.

Das erfindungsgemäße Verfahren wird durch das folgende Beispiel näher erläutert, ohne darauf beschränkt zu sein:

### Beispiel:

Ein Gemisch, bestehend aus 75 Gew.-% Cumol und 25 Gew.-% Cyclohexylbenzol wird bei 132 °C mit Sauerstoff in einem thermostatisierbaren Blasensäulenreaktor oxidiert. Nach einer Oxidationszeit von 3 Stunden enthält das Oxidat laut Analyse mit einem Gaschromatograph (GC-Analyse) 24,5 Gew.-% Cumolhydroperoxid und 8,2 Gew.-% Cyclohexylbenzolhydroperoxid. Dieses Gemisch wird anschließend im Hochvakuum einer Konzentrierung unterworfen, wobei das anfallende Konzentrat 38,3 Gew.-% Cumolhydroperoxid und 13,1 Gew.-% Cyclohexylbenzolhydroperoxid enthält. Das als Destillat anfallende Kohlenwasserstoffgemisch enthält weniger als 1 Gew.-% Peroxid, berechnet als Cumolhydroperoxid. Das Konzentrat wird anschließend bei 50 °C einer einphasigen Spaltung in Gegenwart von 2000 ppm Schwefelsäure, gelöst in Aceton oder in einem Testspaltprodukt bestehend aus Aceton, MEK, Phenol und den Kohlenwasserstoffen gespalten.

Die Spaltausbeute beträgt nach Auswertung der GC-Analyse für Aceton über 95 %, für Cyclohexanon 80,8 % und für Phenol 91,5 %.

## Patentansprüche

1. Verfahren zur Herstellung von Phenol durch Hocksche Spaltung von Arylhydroperoxiden,
dadurch gekennzeichnet,
daß ein Gemisch, das Cumol und Cyclohexylbenzol in einem Gewichtsverhältnis von Cumol zu Cyclohexylbenzol von mindestens 3 : 1 enthält, mit molekularen Sauerstoff enthaltendem Gas oxidiert und dabei entstandenes Cumolhydroperoxid und Cyclohexylbenzolhydroperoxid zu Phenol, Aceton und Cyclohexanon gespalten wird.

2. Verfahren nach Anspruch 1,
dadurch gekennzeichnet,
daß das Cumol/Cyclohexylbenzol-Gewichtsverhältnis im Gemisch von 5 : 1 bis 9 : 1 beträgt.

3. Verfahren nach zumindest einem der vorherigen Ansprüche,
dadurch gekennzeichnet,
daß die Oxidation mit Luft oder Sauerstoff als molekularen Sauerstoff enthaltendem Gas erfolgt.

4. Verfahren nach zumindest einem der vorherigen Ansprüche,
dadurch gekennzeichnet,
daß die Oxidation bei Temperaturen von 100 °C bis 140 °C erfolgt.

5. Verfahren nach zumindest einem der vorherigen Ansprüche,
dadurch gekennzeichnet,
daß das Oxidat vor der Spaltung destillativ durch Abtrennen von Cumol oder Cyclohexylbenzol oder beidem aufkonzentriert wird.

6. Verfahren nach Anspruch 5,
dadurch gekennzeichnet,
daß abgetrenntes Cumol oder Cyclohexylbenzol oder beides zur Oxidation zurückgeführt wird.

7. Verfahren nach zumindest einem der vorherigen Ansprüche,
dadurch gekennzeichnet,
daß das Spaltgemisch homogen ist.

8. Verfahren nach zumindest einem der vorherigen Ansprüche,
dadurch gekennzeichnet,
daß die Spaltung säurekatalysiert wird.

9. Verfahren nach Anspruch 8,
dadurch gekennzeichnet,
daß als Säure Schwefelsäure verwendet wird.
